# EUROPÄISCHE PATENTSCHRIFT

(11) **EP 2 049 227 B1**
(45) Veröffentlichungstag und Bekanntmachung des Hinweises auf die Patenterteilung: **25.04.2012**
(21) Anmeldenummer: 07786492.4
(22) Anmeldetag: 01.08.2007
(51) Int. Cl.: B01D 46/00, B01D 46/42, A61L 2/20, A61L 2/22

(54) **FILTEREINHEIT**
FILTER UNIT
UNITÉ DE FILTRE

(30) Priorität: 04.08.2006 DE 102006036476
(43) Veröffentlichungstag der Anmeldung: 22.04.2009
(73) Patentinhaber: KHS GmbH, 44143 Dortmund (DE)
(72) Erfinder: SANGI, Daryoush, 20251 Hamburg (DE)
(86) Internationale Anmeldenummer: PCT/EP2007/006806
(87) Internationale Veröffentlichungsnummer: WO 2008/014992

(56) Entgegenhaltungen:
- EP-A- 0 815 919
- DE-A1- 2 631 260
- JP-A- 8 021 642
- JP-A- 63 274 426
- US-A- 4 707 167

## Beschreibung

Die Erfindung betrifft eine Filtereinheit zur Reinigung von Luft mit wenigstens einer Filterschicht sowie ein Verfahren zur Sterilisation einer derartigen Filtereinheit.

Filtereinheiten zur Reinigung von Luft sind in unterschiedlichen Ausgestaltungen bekannt. In Fällen, in denen eine besonders hohe Filterleistung, insbesondere im Hinblick auf die Entfernung von Partikeln ab einer Größe von 0,1 µm, benötigt wird, kommen sogenannte HEPA-Filter zum Einsatz. Diese ermöglichen es, Schwebstoffe und andere Partikel in der zu filternden Luft über einen weiten Größenbereich zu entfernen, wobei es Ausgestaltungen gibt, welche Partikel ab einer Größe von 0,1 µm zu 99,999 % aus der Umgebungsluft entfernen. Eine derartige Filterleistung wird zur Erzeugung und Erhaltung von Rein- und Reinstraum-Bedingungen benötigt, sei es zur Chip-Produktion, in Operationsräumen oder für aseptische Anlagen zur Behandlung von Behältern - wie beispielsweise Flaschen oder Dosen- oder zur Füllung derartiger Behälter.

Bei den letzten beiden Anwendungsfällen muss eine aseptische Atmosphäre aufrecht erhalten werden, d.h. schädliche Keime, Sporen und dergl., müssen möglichst vollstandig aus der Umgebungsluft ferngehalten werden, wozu zweckmäßigerweise die zugeführte Luft bereits durch Filterung von derartigen Substanzen befreit wird.

Gelegentlich kann es nötig werden, derartige Filter zu sterilisieren, beispielsweise um ihre Filterleistung aufrecht zu erhalten oder im Falle von Wartungs- oder Reparaturarbeiten.

Zur Sterilisation derartiger Filter kommen verschiedene Verfahren zum Einsatz, unter anderem die Behandlung mit chemischen Desinfektionsmitteln oder die Bestrahlung mit UV-Licht. Zur chemischen Desinfektion kann beispielsweise Wasserstoffperoxid oder Formaldehyd verwendet werden. Eine derartige Aufbringung von H2O2 ist aus der EP 0 815 919 A2 bekannt. Hierbei wird das für die Filtersterilisation vorgesehen H2O2 In einem separaten Behälter vernebelt, über einen Leitungsweg zum Filter geleitet und auf die Filteraberfläche aufgebracht. Nachfolgend wird heiße Luft über den Filter geführt und das H2O2 aktiviert und/oder getrocknet.

Eine weiteres bekanntes Verfahren bzw. Vorrichtung zeigt die JP 63274426, bei der Heizelemente vorgesehen werden, die eine Keimreduktion ermöglichen. Die bekannten Sterilisationsverfahren für derartige Filtersysteme haben eine Reihe von Nachteilen. Viele chemische Desinfektionsmittel, wie z.B. Formaldehyd, sind übeiriechend, krebserregend oder auf sonstige Art und Weise gesundheitsschädlich, was ihre Anwendung aufwändlg, teuer und risikoreich macht Zur Bestrahlung mit UV-Licht sind aufwändige Bestrahlungseinrichtungen nötig. Bei der Verwendung externer Bestrahlungseinrichtungen müssen die Filterelemente in unsterilisiertem Zustand aus ihrem Gehäuse entnommen werden, was je den, In den Filtern enthaltenen Filterrückständen gefährlich sein kann. Außerdem ist der Filter während derartiger Arbeiten nicht funktionsfähig. Die JP 08021642 zeigt eine derartige Filtereinrichtung für eine Klimaanlage, bei der eine Velzahl von Infrarotstrahlern auf die äußere Filteroberfläche gerichtet ist. Die Integration entsprechender Bestrahlungseinrichtungen ist häufig unwirtschaftlich.

Aufgabe der Erfindung ist es, eine Filtereinheit sowie ein Verfahren zur Sterilisation einer derartigen Filterfeinheit zur Verfügung zu stellen, das eine sichere und erfolgreiche Sterilisatlon des Filters ermöglicht und gleichzeitig zu wirtschaftlichen Kosten realisierbar ist.

Die Erfindung erreicht dies durch eine Filtereinheit gemäß Patentanspruch 1 sowie durch ein Verfahren zur Sterilisation einer derartigen Filterelnheit gemäß Anspruch 4.

Die eigentliche Filtersterilisation geschieht bei dem erfindungsgemäßen Verfahren mittels eines dampfförmigen oder flüssigen Sterillsatonsmittels, das durch Zuführung einer bestimmten Wärmemenge T, die es über eine Aktivierungstemperatur erhitzt, aktivierbar ist. Vorzugsweise handelt es sich bei dem Sterilisationsmedium um Wasserstoffperoxid (H₂O₂).

Dazu wird das Sterilisationsmittel in die Filtereinheit bzw. je nach deren Aufbau, in oder auf die Filterschichten, gebracht und Im Anschluss durch Zuführung von heißer Luft über seine Aktivierungstemperatur erhitzt Auf diese Weise wird eine Zerfallsreaktion gestartet, bei der unter anderem freie Radikale entstehen, die neben weiteren Reaktionen Im Inneren des Filters vorhandene Mikroorganismen, Keime und dergl., abtöten. Am Ende der Reaktion bleiben dann im Wesentlichen Wasser und einige Zerfallsprodukte übrig.

Bei der erfindungsgemäßen Filtereinheit ist hierzu eine Helzelnrichtung zum Beheizen der zu filternden Luft vorgesehen. Diese ermöglicht es, die Luft unmittelbar in der Filtereinheit auf die zur Aktivierung nötige Temperatur zu erhitzen, was eine überraschend einfache und kostensparende Konstruktion ermöglicht.

Durch derartige Filtereinheiten mit integrierter Heizeinrichtung lassen sich diverse Vorteile in der Prozessführung bei mit derartigen Filtereinheiten ausgestatteten Anlagen erzielen. So lässt sich neben der beschriebenen einfachen Sterilisierung der Filter selbst, temperierte gefilterte Luft unmittelbar und einfach der Anlage zuführen. Durch die Integration beispielsweise elektrisch beheizbarer Heizelemente ist eine einfach Steuerung der Temperatur möglich. Aufwändige zusätzliche Heizeinrichtungen im Inneren der Reinräume oder externe Heizeinrichtungen mit Transporttemperaturverlusten können entfallen. Die Heizelemente befinden sich außerhalb der Reinräume, was eine Wartung, Reparaturen oder den Austausch derartiger Elemente stark vereinfacht, da ein Zugriff in das Innere des Reinraumes nicht erfolgt.

Zusätzlich können derartige Filterelemente auch zur Unterstützung von Sterilisationsprozessen im Inneren des Reinraumes dienen, wenn diese mit einem ähnlichen Verfahren, wie der Filter selber, also beispielsweise durch Benutzung von H₂O₂ sterilisiert werden. In diesem Fall wird das Innere des Reinraumes mit einer ausreichenden Menge an flüssigem und/oder dampfförmigem Sterilisationsmittel versorgt und dieses durch Zuführung einer ausreichenden Menge temperierter Luft, die, wie beschrieben, durch die erfindungsgemäßen Filtereinheiten einfach und präzise erzeugt werden kann, aktiviert, so dass die Reaktion auch im Inneren des Reinraumes abläuft.

Diese und weitere Ausgestaltungen der Erfindung ergeben sich aus den Unteransprüchen, die, genau wie die Haupt- und Nebenansprüche, zum Gegenstand der Beschreibung gemacht werden.

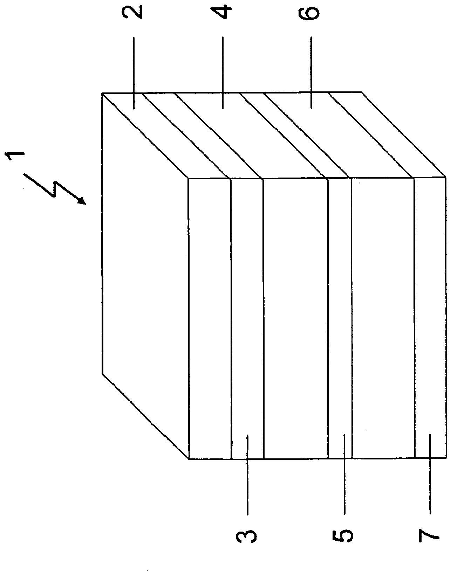

Weitere Vorteile der Erfindung ergeben sich aus der beigefügten Zeichnung, die in ihrer einzigen Abbildung eine schematische dreidimensionale Ansicht einer erfindungsgemäßen Filtereinheit zeigt.

Eine allgemein mit 1 bezeichnete Filtereinheit zur Reinigung von Luft in flächiger Schichtbauweise ist in der Figur näher dargestellt. Sie ist in einem nicht näher dargestellten Gehäuse angeordnet und mit Luftzu- und Abführungen versehen oder mit ihrer Unterseite unmittelbar an einem mit Luft zu versorgenden Reinraum befestigt.

An der Luftzuführungsseite der Filtereinheit 1 ist eine vorgeschaltete Grobfilterschicht 2 angeordnet, die zur Entfernung grober Partikel und Verunreinigung aus der angesaugten Luft dient, um die dahinter liegenden eigentlichen Filterschichten zu entlasten. Darunter angeordnet befindet sich ein steuerbares Gebläse 3, das für den gewünschten Luftstrom durch die Filtereinheit 1 sorgt. Alternativ kann das Gebläse auch außerhalb der Filtereinheit 1 angeordnet sein.

Im Anschluss daran befindet sich eine elektrische Heizeinrichtung 4.

Sie kann beispielsweise in herkömmlicher Lamellenbauweise aufgebaut sein und weist eine der Filterleistung der Einheit angepasste Nennheizleistung auf. Für eine Filterleistung von 1.500 m³/Stunde und eine Erwärmung des Luftstromes auf 80°C hat sich eine Heizleistung der Heizeinrichtung 4 von etwa 36 kW als zweckmäßig erwiesen.

Im Anschluss an die Heizeinrichtung 4 befindet sich eine erste HEPA (High Efficiency Particulate Air) Filterschicht 5 bzw., je nach Anwendung, eine ULPA-Filterschicht z.B. der Klasse U16, die die eigentliche Filterung von Schwebstoffen, Mikroorganismen und dergl. ermöglicht. Hieran schließt sich eine Sterilisationsschicht 6 an, mittels derer flüssiges oder dampfförmiges H₂O₂ in die Filterschicht 5 eingebracht werden kann. Im Anschluss an die Sterilisationsschicht 6 ist eine weitere HEPA- bzw. ULPA-Filterschicht 7 angeordnet, die ebenfalls von der Sterilisationsschicht 6 mit flüssigem und/oder dampfförmigem H₂O₂ versorgt werden kann. In Ausgestaltung der Erfindung ist es auch möglich, nur eine der beiden Filterschichten 5 bzw. 7 zu verwenden oder weitere Filterschichten hieran anzuschließen.

Im Normalbetrieb wird durch das steuerbare Gebläse 3 der nötige Frischluftstrom angesaugt, durch die regelbare Heizeinrichtung 4 auf die nötige Temperatur erwärmt und im Anschluss durch die Filterschichten 5 und 7 gereinigt, so dass am Ende ein hochreiner Luftstrom im angeschlossenen Raum zur Verfügung steht, der, je nach Ausgestaltung der Filterschichten, zu 99,9 % frei von Staubpartikeln oder Mikroorganismen größer als 0,1 bis 0,3 µm ist bzw. durch Verwendung noch leistungsfähiger ULPA-Filterschichten zu 99,999 % frei von den genannten Partikeln, Viren, Bakterien, Milbeneiern und Ausscheidungen, Pollen und dergl. ist.

Soll beispielsweise in einem regelmäßigen Sterilisationszyklus oder für außerplanmäßige Wartungsarbeiten und dergl. der Filter selbst sterilisiert werden, geschieht das mit dem erfindungsgemäßen Verfahren wie folgt:

Zunächst wird von der Sterilisationsschicht 6 flüssiges bzw. dampfförmiges H₂O₂ in die beiden Filterschichten 5 und 7 in ausreichender Menge eingebracht. Hierzu kann zeitweise das Gebläse 3 deaktiviert werden. Ist eine ausreichende Sättigung von H₂O₂ in den Filterschichten erreicht, wird die Heizeinrichtung 4 aktiviert und mittels des Gebläses 3 heiße Luft durch die H₂O₂ enthaltenden Filterschichten geleitet. Hierdurch wird das Sterilisationsmedium über seine Aktivierungstemperatur hinaus erhitzt, so dass ein Zerfallsprozess beginnt, bei dem als Zwischenprodukte unter anderem freie Radikale entstehen, die für eine Zersetzung der enthaltenen Mikroorganismen und dergl. sorgen. Nach Ablauf der Reaktion, für deren Dauer nach der Aktivierung das Gebläse 3 in Ausgestaltung wieder ausgeschaltet werden kann, bleiben als Reaktionsprodukte im Wesentlichen Wasser sowie einige Zerfallsreste über. Durch Aktivierung des Gebläses 3, ggf. in Kombination mit der Aktivierung der Heizeinrichtung 4, können daraufhin die Filterschichten 5 und 7 getrocknet und die überschüssigen Zerfallsprodukte ausgeblasen werden.

Alternativ kann das Gebläse 3 hierzu auch in entgegengesetzter Förderrichtung betrieben werden, um den angeschlossenen Reinraum nicht zu belasten. Dies ist unnötig, sofern die Filtersterilisation im Rahmen eines gemeinsamen Sterilisationszyklus mit dem angeschlossenen Reinraum erfolgt.

Die Erfindung ist nicht auf das vorstehende Ausführungsbeispiel beschränkt, sondern kann in vielfältiger Hinsicht abgewandelt werden, ohne den Grundgedanken zu verlassen. So ist beispielsweise die Filtergeometrie nicht auf die beschriebene Stapelbauweise beschränkt, sondern kann auch bei zylinderförmigen Filteranordnungen oder Radialfiltern, bei denen die Filterschichten koaxial zueinander angeordnet sind, verwendet werden. Auch die Anzahl und Anordnung der Filterschichten kann je nach Anforderungen verändert werden, wobei nicht alle Filter zwangsläufig sterilisierbar ausgestaltet sein müssen. Auch können neben H₂O₂ unter Umständen andere thermisch aktivierbare flüssige und/oder dampfförmige Sterilisationsmittel verwendet werden.

### Bezugszeichenliste

- 1: Filtereinheit
- 2: Grobfilterschicht
- 3: Gebläse
- 4: Heizeinrichtung
- 5: erste Filterschicht
- 6: Sterilisationsschicht
- 7: zweite Filterschicht

## Patentansprüche

1. Filtereinheit (1) zur Reinigung von Luft mit wenigstens zwei Filterschlchten (5,7), wobei in der Filtereinheit (1) zusätzlich eine Integrierte Heizeinrichtung (4) zum Beheizen der Luft vorgesehen ist wobei eine Sterilisationsschicht (6) zur Einbringung eines dampfförmigen oder flüssigen Sterilisationsmittels auf wenigstens eine Filterschicht (5) vorgesehen ist und die Filtereinheit (1) mit einem Gebläse (3) verbunden ist,
**dadurch gekennzeichnet, dass**
die wenigstens zwei Filterschichten (5, 7) Schwebstofffilter sind und die Sterillsationsschicht (6) zwischen den Filterschichten (5, 7) angeordnet ist, wobei mittels der Sterilisationsschicht (6) flüssiges oder dampfförmiges H₂O₂ in die beiden Filterschichten (5, 7) eingebracht werden kann, und wobei die Heizeinrichtung (4) in Strömungsrichtung vor dem ersten Schwebstofffilter angeordnet ist.

2. Filtereinheit nach Anspruch 1, **dadurch gekennzeichnet, dass** die in Strömungsrichtung zweite Filterschicht (7), einen höheren Partikelabscheidegrad als die erste Filterschicht (5) aufweist.

3. Filtereinheit nach Anspruch 1 oder 2, **dadurch gekennzeichnet, dass** in Strömungsrichtung vor der Heizeinrichtung (4) und vor dem ersten Schwebstofffilter ein Grobfilter (2) angeordnet ist.

4. Verfahren zur Sterilisation einer Filtereinheit, **dadurch gekennzeichnet, dass** eine Filtereinheit nach einem der Ansprüche 1 bis 3 vorgesehen ist und ein dampfförmiges und/oder flüssiges Sterilisationsmittel in die Filtereinheit (1) und/oder in die wenigstens eine Filterschicht (5,7) eingebracht wird, und dass im Anschluss das Sterilisationsmittel mittels eines gasförmigen Mediums mit einer Temperatur oberhalb einer Aktivierungstemperatur des Sterillsationsmittels aktiviert wird, wobei das gasförmige Medium mittels der Innerhalb der Filtereinheit (1) vorgesehenen Heizeinrichtung (4) erwärmt wird, wobei als Sterilisationsmittel H₂O₂ und als gasförmiges Medium Luft verwendet wird.

5. Verfahren nach Anspruch 4, **dadurch gekennzeichnet, dass** das Gebläse (3) während der Einbringung des Sterilisationsmittels mindestens zeitweise deaktiviert wird.

## Claims

1. Filter unit (1) for cleaning air, comprising at least two filter layers (5, 7), wherein an integrated heating device (4) for heating the air is additionally provided in the filter unit (1), wherein a sterilisation layer (6) for introducing a sterilising agent in vapour or liquid form is provided on at least one filter layer (5) and the filter unit (1) is connected to a fan (3), **characterised in that** the at least two filter layers (5, 7) are particle filters and the sterilisation layer (6) is arranged between the filter layers (5, 7), wherein H₂O₂ in liquid or vapour form can be introduced into the two filter layers (5, 7) by means of the sterilisation layer (6), and wherein the heating device (4) is arranged upstream of the first particle filter, as seen in the flow direction.

2. Filter unit according to claim 1, **characterised in that** the second filter layer (7), as seen in the flow direction, exhibits a higher degree of particle separation than the first filter layer (5).

3. Filter unit according to claim 1 or 2, **characterised in that** a coarse filter (2) is arranged upstream of the heating device (4) and upstream of the first particle filter, as seen in the flow direction.

4. Method for sterilising a filter unit, **characterised in that** a filter unit according to one of claims 1 to 3 is provided and a sterilising agent in vapour and/or liquid form is introduced into the filter unit (1) and/or into the at least one filter layer (5, 7), and **in that** the sterilising agent is then activated by means of a gaseous medium at a temperature above an activation temperature of the sterilising agent, wherein the gaseous medium is heated by means of the heating device (4) provided within the filter unit (1), wherein H₂O₂ is used as the sterilising agent and air is used as the gaseous medium.

5. Method according to claim 4, **characterised in that** the fan (3) is deactivated at least temporarily during the introduction of the sterilising agent.

## Revendications

1. Unité de filtre (1) pour le nettoyage de l'air avec au moins deux couches de filtre (5, 7), sachant que dans l'unité de filtre (1), un dispositif de chauffage (4) intégré est en outre prévu pour le chauffage de l'air, sachant qu'une couche de stérilisation (6) est prévue pour l'introduction d'un moyen de stérilisation liquide ou sous forme de vapeur sur au moins une couche de filtre (5) et l'unité de filtre (1) est reliée à une soufflante (3),
**caractérisée en ce qu'**
au moins les deux couches de filtre (5, 7) sont des filtres pour matières en suspension dans l'air et la couche de stérilisation (6) est disposée entre les couches de filtre (5, 7), sachant que du H₂O₂ liquide ou sous forme de vapeur peut être introduit dans les deux couches de filtre (5, 7) à l'aide de la couche de stérilisation (6), et sachant que le dispositif de chauffage (4) est disposé dans le sens d'écoulement avant le premier filtre pour matières en suspension dans l'air.

2. Unité de filtre selon la revendication 1, **caractérisée en ce que** la seconde couche de filtre (7) dans le sens d'écoulement présente un degré de séparation de particules supérieur à celui de la première couche de filtre (5).

3. Unité de filtre selon la revendication 1 ou 2, **caractérisée en ce qu'**un filtre grossier (2) est disposé dans le sens d'écoulement avant le dispositif de chauffage (4) et avant le premier filtre pour matières en suspension dans l'air.

4. Procédé de stérilisation d'une unité de filtre, **caractérisé en ce qu'**une unité de filtre selon l'une quelconque des revendications 1 à 3 est prévue et un moyen de stérilisation liquide et/ou sous forme de vapeur est introduit dans l'unité de filtre (1) et/ou dans au moins une couche de filtre (5, 7), et **en ce que** par la suite, le moyen de stérilisation est activé à l'aide d'un agent gazeux à une température supérieure à une température d'activation du moyen de stérilisation, sachant que l'agent gazeux est chauffé à l'aide du dispositif de chauffage (4) prévu dans l'unité de filtre (1), sachant que du H₂O₂ est utilisé comme moyen de stérilisation et de l'air est utilisé comme agent gazeux.

5. Procédé selon la revendication 4, **caractérisé en ce que** la soufflante (3) est désactivée au moins temporairement pendant l'introduction du moyen de stérilisation.
